Europäisches Patentamt

European Patent Office

Office européen des brevets

(19)

(11) Veröffentlichungsnummer: **0 106 857 B1**

(12) **EUROPÄISCHE PATENTSCHRIFT**

(45) Veröffentlichungstag der Patentschrift:
**17.09.86**

(21) Anmeldenummer: **83901185.5**

(22) Anmeldetag: **21.04.83**

(86) Internationale Anmeldenummer:
**PCT/CH 83/00050**

(87) Internationale Veröffentlichungsnummer:
**WO 83/03884 (10.11.83** Gazette **83/26)**

(51) Int. Cl.⁴: **F 17 B 1/26,** B 65 D 90/30,
C 12 M 1/00

(54) **FLEXIBLE ABDECKUNG FÜR EINEN GASSPEICHER.**

(30) Priorität: **22.04.82 CH 2434/82**

(43) Veröffentlichungstag der Anmeldung:
**02.05.84 Patentblatt 84/18**

(45) Bekanntmachung des Hinweises auf die Patenterteilung:
**17.09.86 Patentblatt 86/38**

(84) Benannte Vertragsstaaten:
**AT BE CH DE FR GB LI LU NL SE**

(56) Entgegenhaltungen:
**EP - A - 0 045 114**
**CH - A - 628 130**
**DE - C - 40 779**
**DE - C - 573 423**
**US - A - 2 269 568**

(73) Patentinhaber: **Biowatt AG, Grossmünsterplatz 1,
CH-8001 Zürich (CH)**

(72) Erfinder: **Siegl, Peter, Gfell, CH-8854 Siebnen (CH)**

(74) Vertreter: **Feldmann, Clarence Paul et al, c/o
Patentanwaltsbüro FELDMANN AG Postfach
Kanalstrasse 17, CH-8152 Glattbrugg (CH)**

## Beschreibung

Die Erfindung bezieht sich auf eine flexible Abdeckung für einen Gasspeicher. Durch die flexible Abdeckung bekommt der Gasspeicher ein variables Nutzvolumen, bei ansonsten starren Seitenwänden. Es lassen sich auf diese Weise relativ billige Gasspeicher, kleinerer bis mittlerer Grösse herstellen, wie sie unter anderem bei sogenannten Biogasanlagen benötigt werden.

Ein solcher Gasspeicher kann entweder räumlich getrennt vom Gärbehälter, in dem durch anaerobe Vergärung organischer Substanzen nutzbares Gas erzeugt wird, aufgestellt werden, oder aber auf vorteilhafte Weise in den Gärbehälter integriert sein. Es ist auf einfache Weise möglich, durch eine geeignet angebrachte Belastung der Folie einen konstanten Druck im Gasspeicher aufrecht zu halten, wie dies bei der Nutzung des Gases erforderlich ist.

Flexible Abdeckungen von Gasspeichern, welche mit dem Gärraum von Biogasanlagen kombiniert sind, sind bekannt (zum Beispiel CH-PS 628 130, EU-PS 0 045 114). Sie bestehen aus Folien, welche sich relativ unkontrolliert verformen und damit keine genaue Füllstandsanzeige erlauben. Im weiteren ist die Aufbringung und Lagesicherung der Belastung problematisch, wodurch kein konstanter Druck aufrecht erhalten werden kann. Zur Druckerhöhung ist eine weitere gasdichte Hülle und ein Gebläse erforderlich. Kontrolle und Wartung der gasdichten Folie sind nur sehr beschränkt möglich.

Andere bekannte Gasspeicher mit starrem Deckenteil und als Stulpfolie ausgebildetem Wandteil erfordern eine materialaufwendige Konstruktion und stellen hohe Ansprüche bezüglich Lastverteilung und Führung.

Aus der US-PS 2 269 568, die die Merkmale des Oberbegriffs von Anspruch 1 aufweist, ist ein Druckausgleichspeicher mit einer beweglichen Decke und einem als Stulpfolie ausgebildeten Wandanschluss bekannt. Der ebenfalls als Folie ausgebildete Deckenteil ist durch einen leichten Ring versteift, an dem Ketten und ein gesondertes Gewicht befestigt sind. In entleertem Zustand hängen diese Ketten schlaff und liegen teilweise auf dem Boden des Speichers. Beim Füllen des Speichers werden infolge des steigenden Gasdruckes immer längere Teile der Ketten und zum Schluss auch noch das angehängte Gewicht angehoben. Dies hat zur Folge, dass bei zunehmendem Füllungsgrad zugleich auch der Druck ansteigt. Ein derartiger progressiver Druckanstieg ist für einen Druckausgleichspeicher vorteilhaft, für einen Speicher in dem durch mikrobiologische Gärung erzeugte Gase direkt aufgefangen und gespeichert werden unbrauchbar. Der Druck sollte unabhängig vom Füllstand des Gasspeichers konstant sein, damit Verbraucher direkt, ohne Druckreguliergeräte angeschlossen werden können.

Die Erfindung stellt sich zur Aufgabe, einen Gasspeicher mit variablem Nutzvolumen zu schaffen, dessen flexible Abdeckung bei minimalem Materialaufwand, hohen Ansprüchen an die Betriebssicherheit gerecht wird und die Aufbringung und Verteilung der Belastung für einen konstanten Gasdruck mit einfachen Mitteln erlaubt.

Zusätzlich soll die flexible Abdeckung sowohl bei freistehenden Gasspeichern, als auch für Gasspeicher, die in den Gärbehälter einer Biogasanlage integriert sind, anwendbar sein und an die dort gestellten, spezifischen Anforderungen anpassbar sein. Solche spezifischen Anforderungen sind: Geschlossene, thermische Isolation; Bewegen oder Mischen des Substrates und/oder Durchbrechen einer sich auf dem Substrat bildenden Schwimmdecke.

Die Erfindung löst die gestellte Aufgabe mit einer Abdeckung, die die spezifischen Merkmale des Anspruches 1 aufweist.

Der Aussteifungsring kann entweder unten an der Folie angehängt sein, oder oben auf der Folie aufliegen.

Am Aussteifungsring kann mit einfachen Mitteln ein Laufsteg angebracht werden, der Montage-, Kontroll- und Wartungsarbeiten erleichtert.

In der Zeichnung sind einige Ausführungsbeispiele des Erfindungsgegenstandes vereinfacht dargestellt und deren Wirkungsweise erläutert.

Es zeigen:

Fig. 1 und 2 einen Gasspeicher im Schnitt in vereinfachter Darstellung;

Fig. 3 einen in einen Gärbehälter integrierten Gasspeicher.

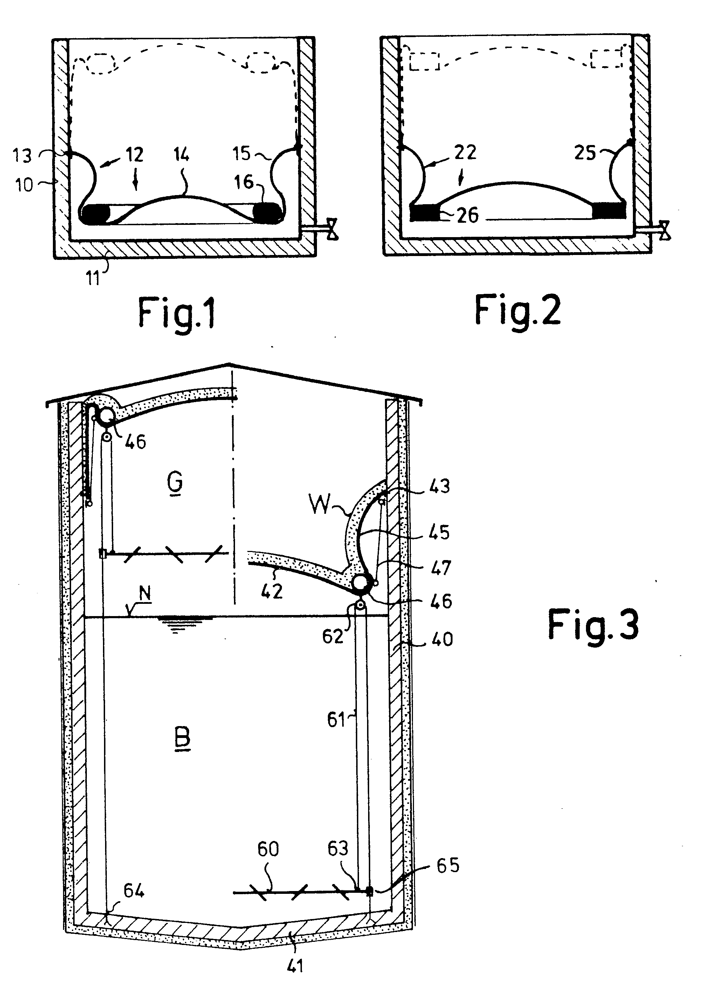

Der in Fig. 1 schematisch dargestellte Gasspeicher umfasst eine starre, gasdichte, kreisringförmige Wand 10 und einen daran anschliessenden, dichten Boden 11.

In etwa halber Höhe ist der Rand einer gasdichten Folie, die in entspanntem Zustand die Form eines nach oben offenen Hohlkegelstumpfes 12 hat, bei 13 an der Wand 10 befestigt. Im Übergangsbereich zwischen dem, den Deckel bildenden Teil 14 der Folie und dem kegelförmigen Wandteil 15 liegt ein Aussteifungsring 16, der so schwer ist, beziehungsweise so beschwert ist, dass sein Gewicht ausreicht, um den gewünschten Gasdruck im Speicher aufrecht zu halten. Durch den Druck ist der Teil 14 in Form ähnlich einer hohlen Kugelkalotte verformt.

Der Rand des Folienteiles 15 ist gasdicht an der Innenseite der Wand 10 befestigt.

In Fig. 1 ist die aus den Teilen 12–16 bestehende Abdeckung in fast entleertem Zustand des Gasspeichers dargestellt. Im oberen Teil der Figur ist die Lage der Abdeckung bei gefülltem Gasspeicher durch unterbrochene Linien dargestellt.

Der Gasspeicher nach Fig. 2 entspricht im Aufbau etwa demjenigen nach Fig. 1, nur ist hier der schwere Aussteifring 26 unten an der Folie 22 angehängt.

Fig. 3 zeigt einen in einen Behälter B für anaerobe Vergärung integrierten Gasspeicher, dessen Abdeckung etwa derjenigen nach Fig. 1 entspricht. Die Seitenwände 40 dieses Behälters B bilden zugleich die Wände für den Gasspeicher G. Der Behälter B ist bis zum Niveau N mit einem in anaerober Gärung befindlichen Substrat gefüllt, das zugleich den unteren Abschluss für den Gasspeicher bildet und denselben mit dem, im Gärungsprozess gebildeten Biogas füllt.

Der Wandteil 45 der Folie 42 ist auch hier wieder in etwa halber Höhe des Gasspeicherteiles G gasdicht an der Wand 40 befestigt.

Als Folienmaterial wird vorzugsweise eine weichmacherfreie Folie verwendet, die bei maximaler Gasdichtigkeit eine bleibende Elastizität gewährleistet.

Zur Aufnahme der aus dem Gasdruck resultierenden Zugkräfte kann entweder eine gewebeverstärkte Folie gewählt, oder eine zweite Folie zur Übernahme der statischen Funktion angeordnet werden.

Gegenüber dem reinen Gasspeicher nach Fig. 1 und 2 besteht ein wesentlicher Unterschied darin, dass die ganze Abdeckung mit einer Wärmeisolationsschicht W versehen ist.

Da für einen optimalen Gärprozess eine gleichbleibende, relativ hohe Temperatur erforderlich ist, muss dazu das Substrat zusätzlich beheizt werden, wozu mindestens ein Teil des erzeugten Gases verwendet wird. Je besser daher die Wärmeisolation, umso weniger Gas muss für die Heizung aufgewendet werden.

Im Bereich der Gasspeicherdecke (zwischen dem Aussteifungsring 46) kann die Isolation W in Form einer, vorzugsweise vorkonfektionierten Matte, welche lose auf die Folie 42 gelegt und entlang des Aussteifungsringes punktweise befestigt ist, haben.

Als Isolationsmaterial kommen sowohl anorganische Faserstoffe, als auch weiche organische Schaumstoffe in Frage.

Im Bereich der beweglichen Stulpmembrane zwischen Behälterwand 40 und Aussteifungsring 46 wird die Isolation W durch eine an der Behälterwand 40 oberhalb der Folienbefestigungsstelle 43 und am Aussteifungsring 46 befestigte, lose über die Folie gelegte Matte aus organischem Weichschaumstoff, gebildet.

Da die Isolation der Behälterwand bis zur Höhe der obersten Speicherstellung mitgeführt wird, muss die Stulpmembran-Isolation nur in dem, nicht an der Wand anliegenden Bereich, wo sie auch keine Zusammendrückung durch den Gasdruck erfährt, wirksam sein. Die Weichschaummatte wird in ihrer Isolationswirkung im entspannten Zustand weder durch den ständigen Wechsel zwischen Zusammendrückung bei oberster Stellung und Entspannung bei unterster Stellung der Abdeckung noch durch die im Verlaufe der Abwicklung entstehende Faltung beeinträchtigt.

In Fig. 3 zeigt die linke Hälfte die Lage der Abdeckung bei vollem, die rechte Hälfte die Lage derselben bei entleertem Behälter. Beide Endlagen werden durch Seile 47 bestimmt.

Die Abdeckung ist mit einem oben auf der Folie aufliegenden Aussteifungsring 46 verbunden. Unten am Aussteifungsring ist mittels Seilen 61 ein Rost 60 angehängt.

Die Seile 61, die den Rost mit dem Aussteifungsring 46 verbinden, sind über Rollen 62 geführt. Ein Ende des Seiles ist bei 63 mit dem Rost 60, das andere Ende bei 64 mit der Bodenplatte 41 verbunden. Diese Anordnung hat den Vorteil, dass der Hub des Rostes 60 grösser ist, als der Hub des Aussteifungsringes 46, und dass zusammen mit der Führung 65 des Rostes am Nachbarseil eine Zentrierungswirkung erreicht wird.

Das Auffüllen und Entleeren des Gasspeichers und somit das Anheben und Senken des Aussteifungsringes 46 geschieht langsam. Da der Hub des Rostes aber grösser ist als derjenige des Aussteifungsringes, ist auch dessen Bewegung entsprechend rascher, was für eine Mischung des Substrates im Behälter B und für das Durchbrechen der sich auf dem Substrat bildenden Schwimmdecke von Vorteil ist. Die Seile 47 bestimmen die oberste und unterste Lage der Abdeckung und damit auch derjenigen des Rostes 60. Zum Zentrieren und Parallelführen der Abdeckung können verschiedene, bekannte Führungssysteme verwendet werden, wie Parallel-Seilführungen, oberhalb, beziehungsweise unterhalb der Abdeckung, oder an der Peripherie der Abdeckung angeordnete Führungselemente.

Man kann auch eine Parallelführung des Aussteifungsringes mittels über Umlenkrollen geführten Seilen bewerkstelligen. Jedes Seil ist dabei an einander diametral gegenüberliegenden Stellen einmal oben und einmal unten am Aussteifungsring befestigt.

Statt die Elemente, welche ein Mischen des Substrates und/oder Durchbrechen einer Schwimmdecke über Seilrollen wie bei Fig. 3 mittelbar mit dem Aussteifungsring zu verbinden, ist es auch möglich, solche Elemente unter Berücksichtigung deren Gewichtes direkt am Aussteifungsring anzubringen.

Die starren Wände des Gasspeichers sind vorzugsweise kreisringförmig aufgebaut, weil dies ein problemloses, stulpenartiges Abwickeln der Folie beim Heben und Senken der Abdeckung gewährleistet.

Die Wand könnte aber auch als angenähert kreisringförmiges Polygon aufgebaut sein. Auch eine Ellipsenform oder angenäherte ellipsenförmige Anordnung ist denkbar. Wichtig ist, dass Ecken vermieden werden, weil dort das stulpenartige Abwickeln der Folie nicht mehr möglich ist.

**Patentansprüche**

1. Flexible Abdeckung für einen Gasspeicher mit konstantem Druck und variablem Nutzvolumen, mit festen Seitenwänden und beweglicher Decke, die einen als Stulpfolie (45) ausgebildeten Wandanschlusteil aufweist, der als integrale, gasdichte Folienkonstruktion in die kugelkalottenförmig gespannte Decke (42) übergeht, wobei im Übergangsbereich zwischen Decke und Stulpfolie ein biege- und torsionssteifer Aussteifungsring (16; 26; 46) angebracht ist, dadurch gekennzeichnet, dass das Gewicht des Aussteifungsringes die für die Aufrechterhaltung eines konstanten Gasdruckes benötigte Belastung darstellt.

2. Flexible Abdeckung nach Anspruch 1 für einen Gasspeicher (G), der in einem Behälter (B) für anaerobe Vergärung integriert ist, dessen Seitenwände (40) nach oben fortgesetzt sind und zugleich die Wände für den Gasspeicher bilden, dadurch gekennzeichnet, dass Wandanschluss- und Deckenteil (45, 42) der Folie mit einer flexiblen Wärmeisolationsschicht (W) versehen sind,

3. Abdeckung nach Anspruch 1, dadurch gekennzeichnet, dass mittels mindestens einmal über Seilrollen (62) geführten Seilen (61) ein Rost (60) am Aussteifungsring angehängt ist.

4. Abdeckung nach Anspruch 1, dadurch gekennzeichnet, dass am Aussteifungsring, an einander diametral gegenüberliegenden Stellen, Seile (61) befestigt sind, die über Rollen (62) geführt sind, und zusammen mit dem Rost (60) eine Parallelführung der Abdeckung bewirken.

5. Abdeckung nach Anspruch 1, dadurch gekennzeichnet, dass die festen Seitenwände (10; 40) des Gasspeichers mindestens angenähert kreisringförmig bis ellipsenförmig angeordnet sind.

**Claims**

1. Flexible covering for a gas reservoir with constant pressure and variable useful volume, having fixed side walls and a movable ceiling, which comprises a wall connection part formed as turnover foil (45) which merges as an integral, gas-tight foil construction into the ceiling (42) which is stretched in the form of a section of a sphere, while a stiffening ring (16; 26; 46) rigid to bending and torsion is provided in the transition region between the ceiling and the turnover foil, characterised in that the weight of the stiffening ring constitutes the loading required for the maintenance of a constant gas pressure.

2. Flexible covering according to claim 1 for a gas reservoir (G) which is integrated in a vessel (B) for anaerobic fermentation, the side walls (40) of which are prolonged upwards and at the same time form the walls of the gas reservoir, characterised in that the wall connection and ceiling parts (45, 42) of the foil are provided with a flexible thermal insulation coating (W).

3. Covering according to claim 1, characterised in that a grid (60) is suspended on the stiffening ring by means of cables (61) guided at least once over cable pulleys (62).

4. Covering according to claim 1, characterised in that cables (61) are secured to the stiffening ring at mutually diametrically opposite points, which cables are conducted over pulleys (62) and together with the grid (60) effect a parallel guidance of the covering.

5. Covering according to claim 1, characterised in that the fixed side walls (10; 40) of the gas reservoir are arranged at least approximately in circular to elliptical form.

**Revendications**

1. Couverture flexible pour récipient à gaz à pression constante et à volume utile variable, à parois latérales fixes et à toit mobile qui présente un élément de raccordement de paroi conçu sous forme de feuille retournée, qui rejoint en tant que structure de feuille solidaire étanche aux gaz le toit (42) tendu en forme de calotte, un anneau de raidissement (16; 26; 46) rigide en flexion et en torsion étant disposé dans la région de transition entre toit et feuille retournée, caractérisée en ce que le poids de l'anneau de raidissement constitue la charge nécessaire au maintien d'une pression constante de gaz.

2. Couverture flexible selon la revendication 1, pour un récipient à gaz (G) qui est intégré à un récipient (B) de fermentation anaérobie dont les parois latérales (40) sont prolongées vers le haut et forment en même temps les parois du réservoir à gaz, caractérisé en ce que les parties de raccordement de paroi et de toit (45, 42) de la feuille sont munies d'une couche flexible d'isolation thermique (W).

3. Couverture selon la revendication 1, caractérisée en ce qu'au moyen de câbles (61) guidés au moins une fois sur des poulies à câbles (62), une grille (60) est accrochée à l'anneau de raidissement.

4. Couverture selon la revendication 1, caractérisée en ce qu'à l'anneau de raidissement sont fixés, à des endroits diamétralement opposés l'un à l'autre, des câbles (61) qui sont guidés sur des poulies (62) et forment avec la grille (60) un guidage parallèle de la couverture.

5. Couverture selon la revendication 1, caractérisée en ce que les parois latérales fixes (10; 40) du réservoir à gaz sont disposées de façon au moins approximativement circulaire à elliptique.

Fig.1

Fig.2

Fig.3